# EUROPÄISCHE PATENTSCHRIFT

(11) **EP 1 567 211 B1**
(45) Veröffentlichungstag und Bekanntmachung des Hinweises auf die Patenterteilung: **28.04.2010**
(21) Anmeldenummer: 03767727.5
(22) Anmeldetag: 02.12.2003
(51) Int. Cl.: A61M 1/36, B01D 29/01

(54) **FILTER**
FILTER
FILTRE

(30) Priorität: 02.12.2002 DE 10256160
(43) Veröffentlichungstag der Anmeldung: 31.08.2005
(73) Patentinhaber: Fresenius HemoCare Deutschland GmbH, 61352 Bad Homburg (DE)
(72) Erfinder: CAVALLINI, Paolo, I-41032 Cavezzo (IT); KOOS, Hakvoort, NL-7827 NW Emmen (NL); MATHIEU, Bernd, 66583 Spiesen (DE)
(74) Vertreter: Herrmann, Uwe
(86) Internationale Anmeldenummer: PCT/EP2003/013596
(87) Internationale Veröffentlichungsnummer: WO 2004/050147

(56) Entgegenhaltungen:
- EP-A- 0 953 361
- WO-A-95/17237
- US-A- 3 506 130
- PATENT ABSTRACTS OF JAPAN vol. 1996, no. 02, 29. Februar 1996 (1996-02-29) & JP 07 267871 A (KAWASUMI LAB INC), 17. Oktober 1995 (1995-10-17)

## Beschreibung

Die vorliegende Erfindung betrifft einen Filter, insbesondere zur Abtrennung von Leukozyten von weiteren Blutbestandteilen, mit einer Außenhülle, mit wenigstens einer Zwischenschicht, die Bestandteil eines Rahmens ist oder einen Rahmen bildet, mit einer Einlasskammer, die mit einem Einlass für das zu filtrierende Medium in Verbindung steht und einer Auslasskammer, die mit einem Auslass für das Filtrat in Verbindung steht, sowie mit einem Filtermaterial, das die Einlasskammer von der Auslasskammer trennt.

Derartige Filter werden beispielsweise eingesetzt, um Blut oder Blutkomponenten vor der Lagerung bzw. Transfusion an den Empfänger von weißen Blutkörperchen zu befreien, da diese unerwünschte Nebeneffekte hervorrufen können. Es besteht daher die Notwendigkeit, vor der Lagerung oder Transfusion des Blutes bzw. seiner Komponenten (z. B. rote Blutkörperchen, Plasma) weiße Blutkörperchen und gegebenenfalls weitere Störstoffe abzutrennen.

Derartige Filter sind in zahlreichen unterschiedlichen Ausführungsformen bekannt. Der in der EP 0 526 678 B2 offenbarte Filter weist eine aus flexiblem Material bestehende Außenhülle auf, die mit einer Zwischenschicht aus zwei weichen Folien in Verbindung steht, die einen Rahmen bilden. In dem Rahmen ist das Filtermaterial aufgenommen, das die Auslasskammer des Filters von der Einlasskammer trennt. Das Filtermaterial ist in den durch die Folien gebildeten Rahmen eingeschweißt und bewirkt auf diese Weise, dass das zu filtrierende Blut bzw. die zu filtrierenden Blutkomponenten ausschließlich durch die Filterfläche strömen müssen, um in die Auslasskammer zu gelangen.

Aus der WO 01/91880 A1 ist eine Ausführungsform eines Filters bekannt, bei dem die Außenhülle aus zwei Folien besteht, die unmittelbar miteinander verschweißt sind. Eine Zwischenschicht ist nicht vorgesehen. Der Filter weist eine innenliegende und eine außenliegende Schweißnaht auf, wobei die außenliegende Schweißnaht die die Außenhülle bildenden Folien unmittelbar miteinander verbindet und die innenliegende Schweißnaht das Filtermaterial mit der Außenhülle verbindet. Zwischen beiden Schweißnähten befindet sich ein nicht verschweißter, Filtermedium aufweisender Bereich, der aufgrund des Fehlens einer Schweißnaht nachgiebig ist und mittels dessen Beschädigungen bei der Zentrifugation weitgehend vermieden werden können. Der aus der WO 01/91880 A1 bekannte Filter weist den Nachteil auf, dass dieser auf beiden gegenüberliegenden Außenflächen der Außenhülle Stutzen zum Einlass des Blutes bzw. zum Auslass des Filtrats aufweist, wodurch zu beiden Seiten des Filters eine flache Auflage, die zum Zwecke der Zentrifugation erwünscht wäre, verhindert wird.

Bei der aus der EP 0 526 678 B2 bekannten Filtervorrichtung besteht ein Nachteil darin, dass im Bereich des das Filtermaterial einfassenden Rahmens Toträume entstehen, in denen kein Blut filtriert werden kann. Dies ist deshalb unerwünscht, weil zum einen das vorhandene Filtervolumen nicht vollständig ausgenutzt wird. Zum anderen besteht ein Nachteil darin, dass sich in den Toträumen wertvolle Blutkomponenten ansammeln können, die nach der Beendigung der Filtration nicht oder nur schwer zurückgewonnen werden können. Der damit einhergehende Verlust an nicht zurückgewonnenen Blutkomponenten ist unerwünscht.

Es ist daher die Aufgabe der vorliegenden Erfindung, einen Filter der eingangs genannten Art dahingehend weiterzubilden, dass dieser die Möglichkeit einer einfachen Zentrifugation eröffnet und dass die Wahrscheinlichkeit für das Auftreten von Toträumen verringert wird.

Diese Aufgabe wird durch einen Filter gemäß Patentanspruch 1 gelöst. Danach ist das Filtermaterial zwischen der Außenhülle und der Zwischenschicht eingefasst. Durch die Anbindung des Filtermaterials an eine Seite der Außenhülle wird der Vorteil erreicht, dass zumindest auf dieser Seite das Auftreten von Toträumen verhindert werden kann und der vorhandene Raum des Filters entsprechend besser ausgenutzt wird. Des Weiteren kann zumindest eine Auflageseite des Filters ohne Stutzen ausgeführt werden, was für die Zentrifugation von Vorteil ist.

Gegenüber der eingangs genannten EP 0 526 678 B2 ergibt sich aufgrund der erfindungsgemäßen Ausführung des Filters des Weiteren der Vorteil, dass der erfindungsgemäße Filter aus drei Schichten bestehen kann, von denen eine die Zwischenschicht und zwei die Außenhülle bilden, während der in der genannten Druckschrift offenbarte Filter aus wenigstens vier Schichten besteht, nämlich aus zwei Zwischenfolien zur Fixierung des Filtermaterials und aus zwei die Außenhülle bildenden Schichten. Der Filter gemäß der vorliegenden Erfindung kann grundsätzlich auch mehr als drei Schichten aufweisen.

Die Zwischenschicht ist Bestandteil des das Filtermaterial einfassenden Rahmens oder bildet den Rahmen und weist den Vorteil auf, dass aufgrund der Anzahl der verschweißten Schichten die Schweißnähte verhältnismäßig weich ausgeführt werden können, wodurch Beschädigungen während der Zentrifugation weitgehend vermieden werden können. Das Filtermaterial erstreckt sich in der durch den Rahmen gebildeten Ausnehmung.

Ist die Zwischenschicht nur Bestandteil eines Rahmens, werden die weiteren Rahmenabschnitte, d. h. die weiteren das Filtermaterial fixierenden bzw. einfassenden Abschnitte, beispielsweise von der Außenhülle gebildet. In einer derartigen Ausgestaltung der Erfindung ist dementsprechend die erfindungsgemäße Anordnung des Filtermaterials zwischen Zwischenschicht und Außenhülle nur bereichsweise vorgesehen. Die erfindungsgemäße Anordnung des Filtermaterials zwischen Zwischenschicht und Außenhülle muss somit nicht in dem gesamten Filterumfangsbereich vorliegen, sondern kann auch nur für Teilbereiche vorgesehen sein, beispielsweise für den Bereich des Auslasses.

Die Form der Zwischenschicht ist beliebig und kann je nach den bestehenden Anforderungen frei gewählt werden. Denkbar ist beispielsweise, die Zwischenschicht in Form eines geraden oder gekrümmten Streifens, U-förmig, halbkreisförmig etc. oder auch umlaufend auszuführen, wodurch ein geschlossener Rahmen entsteht.

Um Beschädigungen während der Zentrifugation zu vermeiden, ist es vorteilhaft, wenn der Filter eine aus flexiblem Material bestehende Außenhülle aufweist.

Die Außenhülle kann aus zwei miteinander verschweißten Teilen, insbesondere Folien, bestehen, wobei das Filtermaterial zwischen einem dieser Teile und der Zwischenschicht eingefasst ist. In bevorzugter Ausgestaltung der Erfindung besteht die Außenhülle und/oder die Zwischenschicht aus PVC.

Besonders vorteilhaft ist es, wenn dass Filtermaterial zwischen der einlassseitigen Außenhülle und der Zwischenschicht eingefasst ist.

Das Filtermaterial kann mit der Außenhülle und der Zwischenschicht verschweißt sein.

In vorteilhafter Ausgestaltung der Erfindung besteht die Zwischenschicht aus flexiblem Material.

Es kann eine erste innenliegende Schweißnaht vorgesehen sein, die das Filtermaterial mit der Zwischenschicht und der Außenhülle verbindet, und eine zweite außenliegende Schweißnaht, die die Zwischenschicht mit der Außenhülle verbindet.

Die innenliegende Schweißnaht fixiert auf diese Weise das Filtermaterial zwischen der Zwischenschicht und der Außenhülle. Die außenliegende Schweißnaht verbindet die Zwischenschicht mit der Außenhülle.

Bei einer derartigen Ausführungsform ist der Randbereich des Filtermaterials vorzugsweise in einem Bereich angeordnet, der sich zwischen beiden Schweißnähten um das Filtermaterial herum erstreckt. Besonders vorteilhaft ist es, wenn die Zwischenschicht einen oder mehrere Durchbrüche aufweist, die den in den Rahmen eingefassten Randbereich des Filtermaterials mit der Ein- oder Auslasskammer verbinden. Derartige Durchbrüche bringen den Vorteil mit sich, dass in diesem Bereich des Filtermaterials kein oder nur verhältnismäßig wenig Medium verbleibt, sondern von dort aus entweder in die Auslasskammer oder zurück in die Einlasskammer strömt und erneut filtriert werden kann. Der Verlust an z. B. Blut oder Blutkomponenten kann auf diese Weise minimiert werden.

Der oder die Durchbrüche der Zwischenschicht sind vorzugsweise in einem Bereich zwischen der ersten und zweiten Schweißnaht angeordnet. Auf diese Weise kann erreicht werden, dass das filtrierte Medium, insbesondere Blut von diesem Bereich ausgehend vorzugsweise in Auslasskammer strömt. Bei entsprechender Ausführung der Durchbrüche ist es ebenfalls möglich, das filtrierte Blut zurück in die Einlasskammer zu führen und es erneut zu filtrieren.

Gemäß einer weiteren Ausgestaltung der vorliegenden Erfindung sind mehrere Zwischenschichten vorgesehen. In diesem Fall weist der Filter vier oder mehr Schichten auf, von denen z. B. zwei die Außenhülle bilden und z. B. zwei als Zwischenschichten ausgeführt sind. Es sind auch mehr als vier Schichten einsetzbar. Das Risiko der Beschädigung während der Zentrifugation wird verringert, wenn mehr Schichten miteinander verschweißt sind. Durch die Anzahl der Zwischenschichten kann die Weichheit und Nachgiebigkeit der Schweißnähte und somit des Filters erhöht werden.

Besonders vorteilhaft ist es, wenn die Außenhülle aus zwei miteinander verschweißten Folien besteht. Diese können unmittelbar miteinander verschweißt sein oder eine oder mehrere Zwischenschichten zwischen sich aufnehmen.

In weiterer Ausgestaltung der vorliegenden Erfindung ist vorgesehen, dass das Filtermaterial in seiner Draufsicht elliptisch ausgeführt ist. Diese Ausgestaltung ermöglicht eine optimale Filternutzung durch gleichmäßige Verteilung des Blutes auf dem Filtermaterial. Des weiteren lässt sich durch eine elliptische Ausführung das Auftreten von Totzonen weitgehend vermeiden.

Der Einlass für das zu filtrierende Medium, beispielsweise für das Konzentrat aus roten Blutkörperchen, kann aus einem mit der Außenhülle verschweißten Stutzen bestehen, der aus zwei rechtwinklig zueinander angeordneten Schenkeln besteht, von denen der mit der Außenhülle verschweißte Schenkel sich im wesentlichen senkrecht von der Außenhülle erstreckt.

Der Auslass kann aus einem mit der Außenhülle und/oder der Zwischenschicht verschweißten zylindrischen Stutzen bestehen, dessen Längsachse sich in einer Ebene erstreckt, die zu der durch das Filtermaterial gebildeten Ebene parallel verläuft und gegenüber dieser versetzt ist. Der Auslassstutzen ist somit "asymmetrisch" angeordnet. Er kann zwischen der Außenhülle und der Zwischenschicht verschweißt sein oder auch unmittelbar zwischen zwei die Außenhülle bildenden Folien.

Besonders vorteilhaft ist es, wenn das Filtermaterial gepresst oder genadelt ist. Eine derartige Ausführung des Filtermaterials verringert das Filtervolumen bei gleichbleibender Filtermasse und erhöht somit die Filterdichte. Dies hat den Vorteil, dass die Stabilität des Filtermediums insbesondere während der Zentrifugation erhöht wird. Des weiteren wird die Rückgewinnung von Blut aus dem Filter aufgrund des geringeren Volumens bei gleichbleibender Filterleistung verbessert.

Weitere Einzelheiten und Vorteile der vorliegenden Erfindung werden anhand eines in der Zeichnung dargestellten Ausführungsbeispiels näher erläutert. Es zeigen:
- Figur 1:: eine Seitenansicht sowie eine Draufsicht auf den erfindungsgemäßen Leukozytenfilter,
- Figur 2:: eine Schnittdarstellung gemäß Schnittlinie A-A in Fig.1
- Figur 3:: eine vergrößerte Darstellung von Detail B sowie Detail C in Figur 2 und
- Figur 4:: unterschiedliche Darstellungen einer weiteren Ausführungsform eines erfindungsgemäßen Filters.

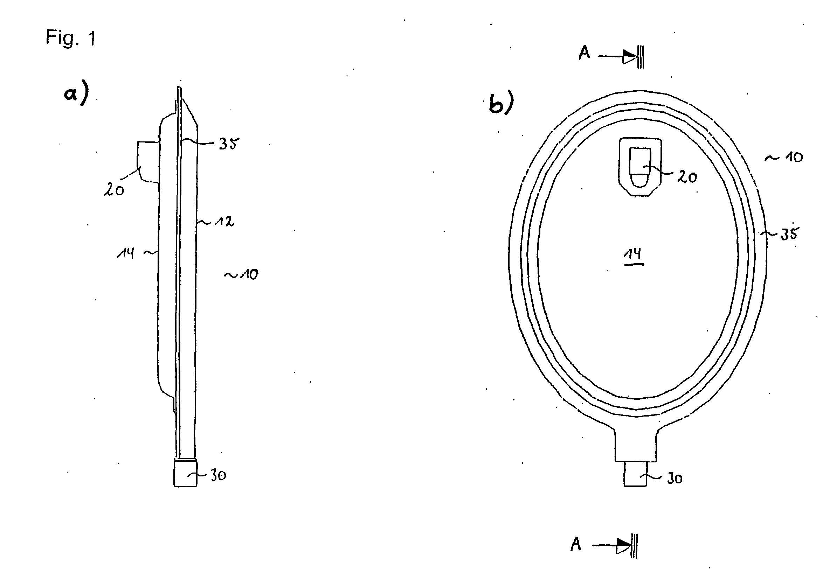

Figur 1a) zeigt in einer Seitenansicht den erfindungsgemäßen Leukozytenfilter 10. Dieser weist eine aus den Folien 12, 14 bestehende Außenhülle auf. Die Folien 12, 14 sind flexibel ausgeführt und bestehen aus PVC. Die Außenfolie 14 ist mit dem Einlassstutzen 20 verschweißt, der zwei rechtwinklig zueinander angeordnete Schenkel aufweist, von denen der mit der Folie 14 verschweißte Schenkel sich senkrecht zu dieser erstreckt, während der andere Schenkel im wesentlichen parallel zu der Außenfolie 14 verläuft.

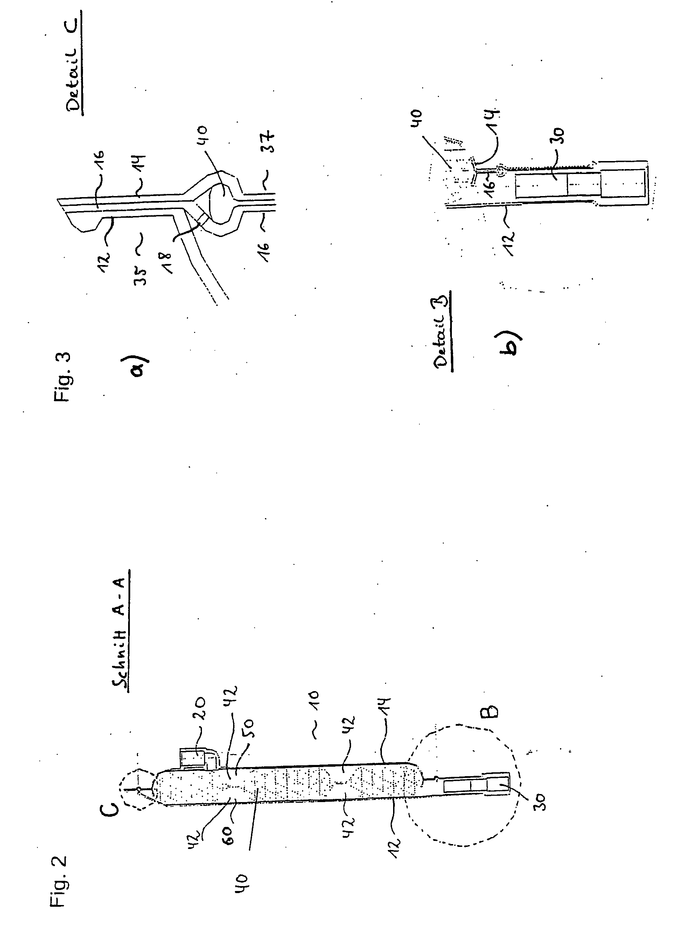

Des Weiteren ist der Auslassstutzen 30 vorgesehen, der im wesentlichen zylindrisch ausgeführt ist. Dieser ist mit der Außenfolie 12 sowie mit einer eine Zwischenschicht bildenden Folie 16 (siehe Figur 3, Detail B) verschweißt.

Die Folien 12, 14 sind an ihrem Umfang mit der Zwischenschicht 16 verschweißt, wodurch eine außenliegende Schweißnaht 35 gebildet wird.

Wie aus Figur 1a) ersichtlich, befindet sich die Längsachse des Stutzens 30 nicht in der Ebene der Schweißnaht 35, sondern in einer Ebene, die dazu parallel verläuft, und gegenüber der Ebene der Schweißnaht 35 versetzt ist.

Aus der Draufsicht auf den Leukozytenfilter gemäß Figur 1b) ergibt sich, dass dieser eine elliptische Form aufweist. Der Einlassstutzen 20 ist nicht im Zentrum des elliptischen Bereiches, sondern gemäß Figur 1 oberhalb davon angeordnet. Die elliptische Ausgestaltung des Filters ermöglicht eine ideale Verteilung des zu filtrierenden Mediums, wodurch der Filter weitgehend ausgenutzt wird. Zudem können Totzonen weitgehend vermieden werden.

Figur 2 zeigt die Schnittdarstellung des Leukozytenfilters gemäß der Schnittlinie A-A in Figur 1. Hieraus wird die Anordnung des Filtermaterials 40 ersichtlich, dass die Einlassseite 50, in die das zu filtrierende Medium nach dem Austritt aus dem Einlassstutzen 20 einströmt, von der Auslassseite 60, in die das Filtrat durch den Filter strömt und durch den Stutzen 30 verlässt, trennt. Das Filtermaterial 40 besteht aus Polybutylenterephthalat - Fasern, die beschichtet sein können. Das Filtermaterial 40 kann mehrschichtig ausgeführt sein.

Bei dem Filtermaterial 40 handelt es sich um ein gepresstes oder genadeltes Filterelement, was durch die Vertiefungen 42 in schematischer Weise angedeutet werden soll. Ein derartiges Filterelement weist den Vorteil auf, dass dessen mechanische Stabilität erhöht wird, was insbesondere zum Zwecke der Zentrifugation erforderlich ist. Ein weiterer Vorteil besteht darin, dass die Rückgewinnung des sich im Filtermaterial befindlichen Mediums erleichtert wird. Die Filterleistung wird durch das verringerte Volumen des Filtermaterials nicht negativ beeinflusst. Die Menge des Filtermaterials bleibt gegenüber einer nicht gepressten bzw. nicht genadelten Ausführungsform identisch.

Figur 3 zeigt die Details B (Fig. 3b)) und C (Fig. 3a)) gemäß Figur 2. Detail B betrifft den Bereich des Auslassstutzens 30, der zwischen der Außenfolie 12 und der mit der Außenfolie 14 verschweißten Zwischenfolie 16 verschweißt ist. Der Auslassstutzen 30 erstreckt sich parallel zu der Längsachse des elliptisch ausgeführten Filters. Wie insbesondere aus Figur 2 ersichtlich, ist der Auslassstutzen 30 an der tiefsten Stelle der Auslasskammer 60 angeordnet, so dass diese vollständig entleert werden kann. Wie insbesondere aus Fig. 2 ersichtlich, lässt sich auf diese Weise eine im Wesentlichen flache Auflage des Filters bilden, die im vorliegenden Fall durch die Außenfolie 12 gebildet wird.

Wie ebenfalls aus Figur 2 und Detail B in Figur 3b) ersichtlich, befindet sich die Mittelachse des zylindrisch ausgeführten Auslassstutzens 30 nicht in der Ebene, die durch das Filtermaterial 40 gebildet wird, sondern ist dazu versetzt ausgeführt.

Aus Detail C (Fig. 3a)) ergibt sich eine vergrößerte Ansicht des oberen Endbereiches des Filters 10. Hier dargestellt ist die Außenfolie 14, das Filtermaterial 40 sowie die Zwischenfolie 16, die mittels einer ersten, innenliegenden Schweißnaht 37 miteinander verschweißt sind. Des weiteren ist die zweite, außenliegende Schweißnaht 35 ersichtlich, die die Folien 12 und 14 mit der Zwischenfolie 16 verbindet. Zwischen diesen Schweißnähten befindet sich der Randbereich des Filtermaterials 40. Wie aus Detail C gemäß Fig. 3a) ersichtlich, weist die Zwischenfolie 16 eine Ausnehmung 18 auf, die den Randbereich des Filtermaterials 40 mit der Auslasskammer verbindet. Dadurch wird erreicht, dass Blut oder Blutbestandteile auch aus diesem Randbereich des Filtermaterials 40 zurückgewonnen werden können. Wie aus Figur 3 ersichtlich, ist das Filtermaterial 40 zwischen der die einlassseitige Außenhülle bildenden Folie 14 und der Zwischenschicht 16 verschweißt. Dadurch ergibt sich der Vorteil, dass der Filter auf der Einlassseite totraumfrei ausgeführt ist, da das Filtermaterial 40 unmittelbar mit der Außenfolie 14 verschweißt ist, wie dies auch aus Figur 2 hervorgeht.

Das folgende Beispiel verdeutlicht den Einfluss der erfindungsgemäßen Durchbrüche der Zwischenschicht sowie der Behandlung des Filtermaterials:

### Beispiel 1:

35 cm² Filterfläche, 26 Schichten von beschichtetem PBT (durchschnittlicher Durchmesser 2 µm, Oberflächendichte 50 g/m²); Ausführung der Schweißnähte gemäß Fig. 1 - 3:
WBC (white blood cells): weniger als 200.000/RCC (red cell concentrate)-Einheit;
Filtrationsdauer: 10-13 Minuten;
RCC-Ausbeute: 93%.

### Beispiel 2:

Der Filter entspricht dem Filter zu Beispiel 1, jedoch im nicht genadelten oder gepressten Zustand des Filtermaterials;
WBC: 200.000 - 400.000;
Filtrationsdauer: 15 - 17 Minuten;
RCC- Ausbeute: 89%.

### Beispiel 3:

Der Filter entspricht dem Filter zu Beispiel 1, jedoch ohne Durchbrüche der Zwischenschicht;
WBC: weniger als 200.000/RCC-Einheit;
Filtrationsdauer: 10 - 13 Minuten;
RCC-Ausbeute: 91 %.

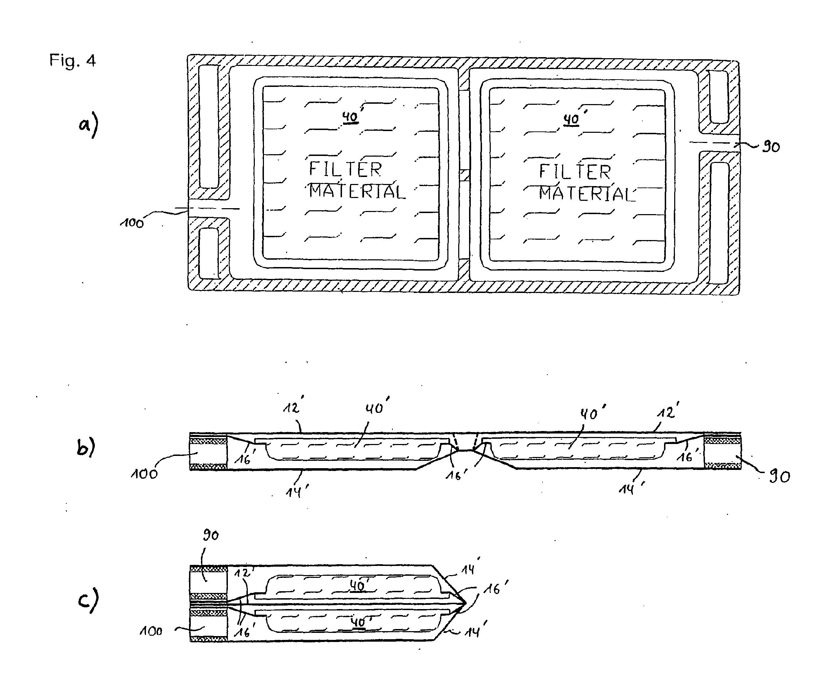

Figur 4 zeigt in unterschiedlichen Ansichten (Fig. 4a), b), c)) ein weiteres Ausführungsbeispiel der vorliegenden Erfindung, in denen die Anbindung des Randbereiches des Filtermaterials an die Zwischenschicht vereinfacht dargestellt ist.

Bei dem Ausführungsbeispiel gemäß Figur 4 sind zwei in Reihe angeordnete Filtermedien 40' vorgesehen. Diese sind in ihren Randbereichen mit einer als Zwischenschicht ausgefüllten Folie 16' verschweißt. Die Folie 16' ist des Weiteren mit der Folie 12' und 14' verschweißt, die die Außenhülle des Filters bilden.

Das zu filtrierende Medium strömt durch den Einlassstutzen 90, der wie auch der Auslassstutzen 100 zylindrisch ausgeführt ist, ein und durchströmt zunächst das rechts dargestellte Filtermaterial 40' von unten nach oben. Von dort wird das Medium durch entsprechende Ausnehmungen auf die Oberseite des links angeordneten Filtermaterial 40' geführt, durchströmt dieses und wird schließlich durch den Auslassstutzen 100 abgezogen.

Die den Außenhüllen bildenden Folien 12', 14' sowie auch die die Zwischenschicht bildende Folie 16' sind aus PVC ausgeführt.

Der in Figur 4 dargestellte Filter ist symmetrisch ausgeführt und kann um seine Mittelachse gefaltet werden, wie dies in Figur 4c) wiedergegeben ist. In diesem Zustand kann der Filter besonders vorteilhaft zentrifugiert werden.

## Patentansprüche

1. Filter, insbesondere zur Abtrennung von Leucozyten von weiteren Blutbestandteilen, mit einer Außenhülle (12, 14) mit wenigstens einer Zwischenschicht (16), die Bestandteil eines Rahmens ist oder einen Rahmen bildet, mit einer Einlasskammer, die mit einem Einlass (20) für das zu filtrierende Medium in Verbindung steht, und einer Auslasskammer, die mit einem Auslass (30) für das Filtrat in Verbindung steht, sowie mit einem Filtermaterial (40), das die Einlasskammer von der Auslasskammer trennt,
**dadurch gekennzeichnet,**
**dass** das Filtermaterial zwischen der Außenhülle und der Zwischenschicht eingefasst ist.

2. Filter nach Anspruch 1, **dadurch gekennzeichnet, dass** die Aussenhülle aus flexiblem Material besteht.

3. Filter nach Anspruch 1 oder 2, **dadurch gekennzeichnet, dass** die Außenhülle aus zwei miteinander verschweißten Teilen, insbesondere Folien, besteht und dass das Filtermaterial zwischen einem dieser Teile und der Zwischenschicht eingefasst ist.

4. Filter nach einem der vorhergehenden Ansprüche, **dadurch gekennzeichnet, dass** das Filtermaterial zwischen der einlassseitgen Außenhülle und der Zwischenschicht eingefasst ist.

5. Filter nach einem der vorhergehenden Ansprüche, **dadurch gekennzeichnet, dass** das Filtermaterial mit der Außenhülle und der Zwischenschicht verschweißt ist.

6. Filter nach einem der vorhergehenden Ansprüche, **dadurch gekennzeichnet, dass** die Zwischenschicht aus flexiblem Material besteht.

7. Filter nach einem der vorhergehenden Ansprüche, **dadurch gekennzeichnet, dass** eine erste innenliegende Schweißnaht vorgesehen ist, die das Filtermaterial mit der Zwischenschicht und der Außenhülle verbindet, und dass eine zweite außenliegende Schweißnaht vorgesehen ist, die die Zwischenschicht mit der Außenhülle verbindet.

8. Filter nach einem der vorhergehenden Ansprüche, **dadurch gekennzeichnet, dass** die Zwischenschicht einen oder mehrere Durchbrüche aufweist, die den in den Rahmen eingefassten Randbereich des Filtermaterials mit der Ein- oder Auslasskammer verbinden.

9. Filter nach Anspruch 7 und 8, **dadurch gekennzeichnet, dass** die Durchbrüche in einem Bereich zwischen der ersten und zweiten Schweißnaht angeordnet sind.

10. Filter nach einem der vorhergehenden Ansprüche, **dadurch gekennzeichnet, dass** mehrere Zwischenschichten vorgesehen sind.

11. Filter nach einem der vorhergehenden Ansprüche, **dadurch gekennzeichnet, dass** das Filtermaterial in seiner Draufsicht elliptisch ausgeführt ist.

12. Filter nach einem der vorhergehenden Ansprüche, **dadurch gekennzeichnet, dass** der Einlass aus einem mit der Außenhülle verschweißten Stutzen besteht, der zwei rechtwinklig zueinander angeordnete Schenkel aufweist, von denen der mit der Außenhülle verschweißte Schenkel sich im wesentlichen senkrecht von der Außenhülle erstreckt.

13. Filter nach einem der vorhergehenden Ansprüche, **dadurch gekennzeichnet, dass** der Auslass aus einem mit der Außenhülle und/oder der Zwischenschicht verschweißten zylindrischen Stutzen besteht, dessen Längsachse sich in einer Ebene erstreckt, die zu der durch das Filtermaterial gebildeten Ebene parallel verläuft und gegenüber dieser versetzt ist.

14. Filter nach einem der vorhergehenden Ansprüche **dadurch gekennzeichnet, dass** das Filtermaterial gepresst oder genadelt ist.

## Claims

1. A filter, in particular for the separation of leucocytes from further blood components, comprising an outer sheath (12, 14), comprising at least one intermediate layer (16) which is a component of a frame or forms a frame, comprising an inlet chamber, which is in communication with an inlet (20) for the medium to be filtered, and an outlet chamber, which is in communication with an outlet (30) for the filtrate, and comprising a filter material (40) which separates the inlet chamber from the outlet chamber,
**characterized in that**
the filter material is encompassed between the outer sheath and the intermediate layer.

2. A filter in accordance with claim 1, **characterized in that** the outer sheath is made of flexible material.

3. A filter in accordance with either of claims 1 or 2, **characterized in that** the outer sheath consists of two parts, in particular foils, which are welded to one another; and **in that** the filter material is encompassed between one of these parts and the intermediate layer.

4. A filter in accordance with any one of the preceding claims, **characterized in that** the filter material is encompassed between the outer sheath on the inlet side and the intermediate layer.

5. A filter in accordance with any one of the preceding claims, **characterized in that** the filter material is welded to the outer sheath and to the intermediate layer.

6. A filter in accordance with any one of the preceding claims, **characterized in that** the intermediate layer consists of flexible material.

7. A filter in accordance with any one of the preceding claims, **characterized in that** a first inwardly disposed welding seam is provided which connects the filter material to the intermediate layer and to the outer sheath; and **in that** a second outwardly disposed welding seam is provided which connects the intermediate layer to the outer sheath.

8. A filter in accordance with any one of the preceding claims, **characterized in that** the intermediate layer has one or more apertures which connect the marginal region of the filter material encompassed in the frame to the inlet chamber or outlet chamber.

9. A filter in accordance with either of claims 7 or 8, **characterized in that** the apertures are arranged in a region between the first and second welding seams.

10. A filter in accordance with any one of the preceding claims, **characterized in that** a plurality of intermediate layers are provided.

11. A filter in accordance with any one of the preceding claims, **characterized in that** the filter material is elliptic in its plan view.

12. A filter in accordance with any one of the preceding claims, **characterized in that** the inlet consists of a stub which is welded to the outer sheath and which has two limbs which are arranged at right angles to one another and of which the limb welded to the outer sheath extends substantially perpendicular from the outer sheath.

13. A filter in accordance with any one of the preceding claims, **characterized in that** the outlet consists of a cylindrical stub which is welded to the outer sheath and/or to the intermediate layer and whose longitudinal axis extends in a plane which extends parallel to the plane formed by the filter material and is offset with respect to it.

14. A filter in accordance with any one of the preceding claims, **characterized in that** the filter material is pressed or needled.

## Revendications

1. Filtre, en particulier pour la séparation de leucocytes d'autres composants du sang, avec une enveloppe extérieure (12, 14) avec au moins une couche intermédiaire (16) qui fait partie d'un cadre ou forme un cadre, avec une chambre d'admission qui est en liaison avec une admission (20) pour le milieu à filtrer, et avec une chambre d'évacuation qui est en liaison avec une évacuation (30) pour le filtrat, et avec un matériau filtrant (40) qui sépare la chambre d'admission de la chambre d'évacuation,
**caractérisé en ce que**
le matériau filtrant est bordé entre l'enveloppe extérieure et la couche intermédiaire.

2. Filtre selon la revendication 1, **caractérisé en ce que** l'enveloppe extérieure est constituée d'un matériau flexible.

3. Filtre selon la revendication 1 ou 2, **caractérisé en ce que** l'enveloppe extérieure est constituée de deux parties, en particulier de feuilles, soudées l'une à l'autre, et **en ce que** le matériau filtrant est bordé entre une de ces parties et la couche intermédiaire.

4. Filtre selon l'une des revendications précédentes, **caractérisé en ce que** le matériau filtrant est bordé entre l'enveloppe extérieure côté admission et la couche intermédiaire.

5. Filtre selon l'une des revendications précédentes, **caractérisé en ce que** le matériau filtrant est assemblé par soudage avec l'enveloppe extérieure et la couche intermédiaire.

6. Filtre selon l'une des revendications précédentes, **caractérisé en ce que** la couche intermédiaire est constituée d'un matériau flexible.

7. Filtre selon l'une des revendications précédentes, **caractérisé en ce qu'**une première soudure située à l'intérieur est prévue qui relie le matériau filtrant avec la couche intermédiaire et l'enveloppe extérieure, et **en ce qu'**une deuxième soudure située à l'extérieur est prévue qui relie la couche intermédiaire à l'enveloppe extérieure.

8. Filtre selon l'une des revendications précédentes, **caractérisé en ce que** la couche intermédiaire présente un ou plusieurs perçages qui relient la zone de bord du matériau filtrant enchâssé dans le cadre avec la chambre d'admission ou d'évacuation.

9. Filtre selon la revendication 7 et 8, **caractérisé en ce que** les perçages sont disposés dans une zone entre la première et la deuxième soudure.

10. Filtre selon l'une des revendications précédentes, **caractérisé en ce que** plusieurs couches intermédiaires sont prévues.

11. Filtre selon l'une des revendications précédentes, **caractérisé en ce que** le matériau filtrant, vu de dessus, est réalisé en une forme elliptique.

12. Filtre selon l'une des revendications précédentes, **caractérisé en ce que** l'admission est constituée d'un embout soudé à l'enveloppe extérieure, qui présente deux branches disposées à angle droit l'une à l'autre, dont la branche soudée à l'enveloppe extérieure s'étend sensiblement verticalement depuis l'enveloppe extérieure.

13. Filtre selon l'une des revendications précédentes, **caractérisé en ce que** l'évacuation est constituée d'un embout cylindrique soudé à l'enveloppe extérieure et/ou à la couche intermédiaire, dont l'axe longitudinal s'étend dans un plan qui s'étend parallèlement au plan formé par le matériau filtrant et est décalé par rapport à celui-ci.

14. Filtre selon l'une des revendications précédentes, **caractérisé en ce que** le matériau filtrant est pressé ou aiguilleté.
